# EUROPEAN PATENT APPLICATION

(11) **EP 3 939 998 A1**
(43) Date of publication of application: **19.01.2022**
(21) Application number: 19888794.5
(22) Date of filing: 29.11.2019
(51) Int. Cl.: C07K 16/28, A61K 39/395, A61P 35/00

(54) **CD40 ANTIBODY PHARMACEUTICAL COMPOSITION AND USE THEREOF**

(30) Priority: 30.11.2018 CN 201811448238
(71) Applicant: Jiangsu Hengrui Medicine Co., Ltd., Lianyungang, Jiangsu 222047 (CN); Shanghai Hengrui Pharmaceutical Co., Ltd, Shanghai 200245 (CN)
(72) Inventor: YANG, Jianjian, Shanghai 200245 (CN); LI, Hao, Shanghai 200245 (CN); LIU, Xun, Shanghai 200245 (CN); JIANG, Jiahua, Shanghai 200245 (CN)
(74) Representative: dompatent von Kreisler Selting Werner - Partnerschaft von Patent- und Rechtsanwälten mbB
(86) International application number: PCT/CN2019/121985
(87) International publication number: WO 2020/108621

(57) **Abstract**

The present invention provides a pharmaceutical composition comprising a CD40 antibody or an antigen-binding fragment thereof and an acetic acid-sodium acetate buffer solution, and a use thereof. The pharmaceutical composition may further contain sugar, a non-ionic surfactant, and other excipients. The pharmaceutical composition of the present invention shows good antibody stability.

SEQUENCE LISTING

<110>  JIANGSU HENGRUI MEDICINE CO., LTD.
       SHANGHAI HENGRUI PHARMACEUTICAL CO., LTD

<120>  CD40 ANTIBODY PHARMACEUTICAL COMPOSITION AND USE THEREOF

<130>  P21412626EP

<140>  PCT/CN2019/121985
<141>  2019-11-29

<150>  CN201811448238.5
<151>  2018-11-30

<160>  18

<170>  PatentIn version 3.5

<210>  1
<211>  112
<212>  PRT
<213>  Mus musculus

<400>  1

Gln Val Gln Leu Gln Gln Pro Gly Ala Asp Leu Val Lys Pro Gly Ala
1               5                   10                  15

Ser Val Lys Met Ser Cys Lys Ala Ser Gly Tyr Ile Leu Thr Thr Tyr
            20                  25                  30

Trp Ile Thr Trp Val Lys Gln Arg Pro Gly Gln Gly Leu Glu Trp Ile
        35                  40                  45

Gly Asp Ile His Pro Gly Ser Gly Ser Thr Lys Tyr Asn Glu Lys Phe
    50                  55                  60

Lys Ser Lys Ala Thr Leu Thr Val Asp Thr Ser Ser Ser Thr Ala Tyr
65                  70                  75                  80

Met Gln Leu Thr Arg Leu Ser Ser Glu Asp Ser Ala Val Tyr Tyr Cys
                85                  90                  95

Ala Arg Arg Asp Tyr Trp Gly Gln Gly Thr Thr Leu Thr Val Ser Ser
            100                 105                 110

<210>  2
<211>  112
<212>  PRT
<213>  Mus musculus

<400>  2

Asp Val Leu Met Thr Gln Ser Pro Leu Ser Leu Pro Val Ser Leu Gly
1               5                   10                  15

Asp Gln Ala Ser Ile Ser Cys Arg Ser Ser Gln Asn Ile Val Asn Ser
            20                  25                  30

Gln Gly Asn Thr Tyr Leu Glu Trp Tyr Leu Gln Lys Pro Gly Glu Ser
        35                  40                  45

Pro Lys Leu Leu Ile Tyr Lys Val Thr Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Ala
                85                  90                  95

Ser Leu Val Pro Trp Thr Phe Gly Gly Gly Thr Lys Leu Glu Ile Lys
            100                 105                 110

<210> 3
<211> 10
<212> PRT
<213> Mus musculus

<400> 3

Gly Tyr Ile Leu Thr Thr Tyr Trp Ile Thr
1               5                   10

<210> 4
<211> 17
<212> PRT
<213> Mus musculus

<400> 4

Asp Ile His Pro Gly Ser Gly Ser Thr Lys Tyr Asn Glu Lys Phe Lys
1               5                   10                  15

Ser

<210> 5
<211> 3
<212> PRT
<213> Mus musculus

<400> 5

Arg Asp Tyr
1

<210>  6
<211>  16
<212>  PRT
<213>  Mus musculus

<400>  6

Arg Ser Ser Gln Asn Ile Val Asn Ser Gln Gly Asn Thr Tyr Leu Glu
1               5                   10                  15


<210>  7
<211>  7
<212>  PRT
<213>  (Mus musculus)

<400>  7

Lys Val Thr Asn Arg Phe Ser
1               5


<210>  8
<211>  9
<212>  PRT
<213>  Mus musculus

<400>  8

Phe Gln Ala Ser Leu Val Pro Trp Thr
1               5


<210>  9
<211>  98
<212>  PRT
<213>  Artificial Sequence

<220>
<223>  human germline heavy chain template IGHV1-2

<400>  9

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Gly Tyr
            20                  25                  30


Tyr Met His Trp Val Arg Gln Ala Pro Gly Gln Gly Leu Glu Trp Met
        35                  40                  45


Gly Trp Ile Asn Pro Asn Ser Gly Gly Thr Asn Tyr Ala Gln Lys Phe
    50                  55                  60


Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg

<210>  10
<211>  100
<212>  PRT
<213>  Artificial Sequence

<220>
<223>  human germline light chain template IGkV2-28

<400>  10

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu Gln Thr Pro
            100

<210>  11
<211>  112
<212>  PRT
<213>  Artificial Sequence

<220>
<223>  hu9E5-H1a

<400>  11

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Trp Ile Thr Trp Val Arg Gln Ala Pro Gly Gln Gly Leu Glu Trp Met
        35                  40                  45

Gly Asp Ile His Pro Gly Ser Gly Ser Thr Lys Tyr Asn Glu Lys Phe
    50                  55                  60

Lys Ser Arg Val Thr Met Thr Val Asp Thr Ser Ile Ser Thr Ala Tyr
65                  70                  75                  80

Met Glu Leu Ser Arg Leu Arg Ser Glu Asp Thr Ala Val Tyr Tyr Cys
                85                  90                  95

Ala Arg Arg Asp Tyr Trp Gly Gln Gly Thr Thr Val Thr Val Ser Ser
            100                 105                 110

<210>  12
<211>  112
<212>  PRT
<213>  Artificial Sequence

<220>
<223>  hu9E5-H1b

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Thr Tyr
            20                  25                  30

Trp Ile Thr Trp Val Arg Gln Ala Pro Gly Gln Gly Leu Glu Trp Met
        35                  40                  45

Gly Asp Ile His Pro Gly Ser Gly Ser Thr Lys Tyr Asn Glu Lys Phe
    50                  55                  60

Lys Ser Arg Val Thr Leu Thr Val Asp Thr Ser Ile Ser Thr Ala Tyr
65                  70                  75                  80

Met Glu Leu Ser Arg Leu Arg Ser Glu Asp Thr Ala Val Tyr Tyr Cys
                85                  90                  95

Ala Arg Arg Asp Tyr Trp Gly Gln Gly Thr Thr Val Thr Val Ser Ser
            100                 105                 110

<210>  13
<211>  112
<212>  PRT
<213>  Artificial Sequence

<220>
<223>  hu9E5-H1c

<400>  13

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ile Leu Thr Thr Tyr
            20                  25                  30

Trp Ile Thr Trp Val Arg Gln Ala Pro Gly Gln Gly Leu Glu Trp Met
        35                  40                  45

Gly Asp Ile His Pro Gly Ser Gly Ser Thr Lys Tyr Asn Glu Lys Phe
    50                  55                  60

Lys Ser Arg Val Thr Leu Thr Val Asp Thr Ser Ile Ser Thr Ala Tyr
65                  70                  75                  80

Met Glu Leu Ser Arg Leu Arg Ser Glu Asp Thr Ala Val Tyr Tyr Cys
                85                  90                  95

Ala Arg Arg Asp Tyr Trp Gly Gln Gly Thr Thr Val Thr Val Ser Ser
            100                 105                 110

<210>  14
<211>  112
<212>  PRT
<213>  Artificial Sequence

<220>
<223>  hu9E5-L1a

<400>  14

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Asn Ile Val Asn Ser
            20                  25                  30

Gln Gly Asn Thr Tyr Leu Glu Trp Tyr Leu Gln Lys Pro Gly Gln Ser
        35                  40                  45

Pro Gln Leu Leu Ile Tyr Lys Val Thr Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ala
                85                  90                  95

Ser Leu Val Pro Trp Thr Phe Gly Gly Gly Thr Lys Val Glu Ile Lys
            100                 105                 110

<210>  15
<211>  112
<212>  PRT
<213>  Artificial Sequence

<220>
<223>  hu9E5-L1b

<400>  15

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Asn Ile Val Asn Ser
            20                  25                  30

Gln Gly Asn Thr Tyr Leu Glu Trp Tyr Leu Gln Lys Pro Gly Gln Ser
        35                  40                  45

Pro Gln Leu Leu Ile Tyr Lys Val Thr Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ala
                85                  90                  95

Ser Leu Val Pro Trp Thr Phe Gly Gly Gly Thr Lys Val Glu Ile Lys
            100                 105                 110

<210>  16
<211>  112
<212>  PRT
<213>  Artificial Sequence

<220>
<223>  hu9E5-L1c

<400>  16

Asp Val Leu Met Thr Gln Ser Pro Leu Ser Leu Pro Val Thr Pro Gly
1               5                   10                  15

Glu Pro Ala Ser Ile Ser Cys Arg Ser Ser Gln Asn Ile Val Asn Ser
            20                  25                  30

Gln Gly Asn Thr Tyr Leu Glu Trp Tyr Leu Gln Lys Pro Gly Gln Ser
        35                  40                  45

Pro Gln Leu Leu Ile Tyr Lys Val Thr Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ala
                85                  90                  95

Ser Leu Val Pro Trp Thr Phe Gly Gly Gly Thr Lys Val Glu Ile Lys
            100                 105                 110

<210> 17
<211> 442
<212> PRT
<213> Artificial Sequence

<220>
<223> hu9E5 HC

<400> 17

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ile Leu Thr Thr Tyr
            20                  25                  30

Trp Ile Thr Trp Val Arg Gln Ala Pro Gly Gln Gly Leu Glu Trp Met
        35                  40                  45

Gly Asp Ile His Pro Gly Ser Gly Ser Thr Lys Tyr Asn Glu Lys Phe
    50                  55                  60

Lys Ser Arg Val Thr Leu Thr Val Asp Thr Ser Ile Ser Thr Ala Tyr
65                  70                  75                  80

Met Glu Leu Ser Arg Leu Arg Ser Glu Asp Thr Ala Val Tyr Tyr Cys
                85                  90                  95

Ala Arg Arg Asp Tyr Trp Gly Gln Gly Thr Thr Val Thr Val Ser Ser
            100                 105                 110

Ala Ser Thr Lys Gly Pro Ser Val Phe Pro Leu Ala Pro Ser Ser Lys
        115                 120                 125

Ser Thr Ser Gly Gly Thr Ala Ala Leu Gly Cys Leu Val Lys Asp Tyr
    130                 135                 140

Phe Pro Glu Pro Val Thr Val Ser Trp Asn Ser Gly Ala Leu Thr Ser
145                 150                 155                 160

Gly Val His Thr Phe Pro Ala Val Leu Gln Ser Ser Gly Leu Tyr Ser
                165                 170                 175

Leu Ser Ser Val Val Thr Val Pro Ser Ser Ser Leu Gly Thr Gln Thr
            180                 185                 190

Tyr Ile Cys Asn Val Asn His Lys Pro Ser Asn Thr Lys Val Asp Lys
        195                 200                 205

Lys Val Glu Pro Lys Ser Cys Asp Lys Thr His Thr Cys Pro Pro Cys
    210                 215                 220

Pro Ala Pro Glu Leu Leu Gly Gly Pro Ser Val Phe Leu Phe Pro Pro
225                 230                 235                 240

Lys Pro Lys Asp Thr Leu Met Ile Ser Arg Thr Pro Glu Val Thr Cys
                245                 250                 255

Val Val Val Asp Val Ser His Glu Asp Pro Glu Val Lys Phe Asn Trp
            260                 265                 270

Tyr Val Asp Gly Val Glu Val His Asn Ala Lys Thr Lys Pro Arg Glu
        275                 280                 285

Glu Gln Tyr Asn Ser Thr Tyr Arg Val Val Ser Val Leu Thr Val Leu
    290                 295                 300

His Gln Asp Trp Leu Asn Gly Lys Glu Tyr Lys Cys Lys Val Ser Asn
305                 310                 315                 320

Lys Ala Leu Pro Ala Pro Ile Glu Lys Thr Ile Ser Lys Ala Lys Gly
                325                 330                 335

Gln Pro Arg Glu Pro Gln Val Tyr Thr Leu Pro Pro Ser Arg Asp Glu
            340                 345                 350

Leu Thr Lys Asn Gln Val Ser Leu Thr Cys Leu Val Lys Gly Phe Tyr
        355                 360                 365

Pro Ser Asp Ile Ala Val Glu Trp Glu Ser Asn Gly Gln Pro Glu Asn
    370                 375                 380

Asn Tyr Lys Thr Thr Pro Pro Val Leu Asp Ser Asp Gly Ser Phe Phe
385                 390                 395                 400

Leu Tyr Ser Lys Leu Thr Val Asp Lys Ser Arg Trp Gln Gln Gly Asn
                405                 410                 415

Val Phe Ser Cys Ser Val Met His Glu Ala Leu His Asn His Tyr Thr
            420                 425                 430

Gln Lys Ser Leu Ser Leu Ser Pro Gly Lys
        435                 440

<210>  18
<211>  219
<212>  PRT
<213>  Artificial Sequence

<220>
<223>  hu9E5 LC

<400>  18

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Asn Ile Val Asn Ser
            20                  25                  30

Gln Gly Asn Thr Tyr Leu Glu Trp Tyr Leu Gln Lys Pro Gly Gln Ser
        35                  40                  45

Pro Gln Leu Leu Ile Tyr Lys Val Thr Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ala
                85                  90                  95

Ser Leu Val Pro Trp Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

## Description

This application claims priority to Chinese patent application CN201811448238.5, filed on November 30, 2018, the contents of which are incorporated herein by its entirety.

### Technical Field

The present disclosure relates to the field of pharmaceutical preparations, and specifically relates to a pharmaceutical composition containing a CD40 antibody and an antigen-binding fragment thereof, and a use as a medicine thereof.

### Background

As one of the glycoproteins expressed on the cell surface, CD40 is a type I membrane intrinsic glycoprotein with a molecular weight of 48 kDa that belongs to the tumor necrosis factor receptor (TNFR) superfamily and plays a crucial role in the immune system. It is expressed in a variety of immune cells, such as B cells, dendritic cells, monocytes and macrophages. When signal transduction is mediated by CD40, specialized antigen presenting cells will be activated. The natural ligand of CD40 is designated CD154 or CD40L and is known to be predominantly expressed in mature T lymphocytes. CD40L-mediated signaling transduction can trigger a number of cellular biological events, including activation and proliferation of immune cells, and production of cytokines and chemokines. CD40 signaling is extremely crucial for T cell-dependent immune responses, especially in the context of tumor environment, where CD40-stimulated dendritic cells are capable of activating tumor-specific effector T cells, which have the potential to eradicate tumor cells.

Expression of CD40 has been found in a variety of normal cells and tumor cells including B lymphocytes. For example, melanoma is one of the tumors with CD40 expression, while 30%-70% of solid tumors also express CD40. Activation of CD40 is known to effectively trigger anti-tumor responses (Tong et al, Cancer Gene Therapy, 2003, 10: 1-13)*,* including immune activation of tumor-specific T cell responses, direct apoptosis of CD40-positive tumors and humoral reaction of ADCC caused by stimulation. Moreover, observed tumor eradication is strongly associated with the occurrence of tumor specific cytotoxic T lymphocytes. Meanwhile, it also should not be ignored that systemic administration of CD40 antibodies generally associates with a variety of side effects such as shock syndrome and cytokine release syndrome (vanMierlo et al, Proc. Natl. Acad. Sci. USA, 2002, 99: 5561-5566).

Currently a number of international pharmaceutical companies are developing monoclonal antibodies against CD40 as described above, which specifically stimulate immune activation, maximizing patients' own immune system response to tumors, thereby achieving the purpose of killing tumor cells. Related patents include CN1198647, CN1369015, CN1582165, CN100430419, CN101014386, CN101237882, CN101289510, CN101490086, CN103842382, CN104918957, WO2002028904, WO2011123489, WO2012149356, WO2013034904, WO2015091853, WO2016196314, WO2017040932 and WO2017004006 etc. So far, anti-CD40 antibodies from Pfizer (relevant products have been licensed to Roche), Alligator and other companies have observed good tumor killing effects in preclinical animal models.

WO2018219327 (PCT/CN2018/089252) provides anti-CD40 antibodies with high affinity, high selectivity and high biological activity for use by stimulating CD40 antibodies.

However, antibody drugs with large molecular weights and complex structures may become unstable due to the tendency of degradation, polymerization or undergoing undesirable chemical modifications. In order to make the antibodies suitable for administration and maintain stability and better performance during storage and subsequent use, research on stable preparations of antibody drugs is of great important. An example of a patent related to CD40 antibody preparation is WO2005063289. For new CD40 antibody, there is still a need to develop pharmaceutical compositions (preparations) comprising CD40 that are more suitable for administration.

### Content of the present invention

The present disclosure provides a pharmaceutical composition comprising a CD40 antibody or an antigen-binding fragment thereof, and a buffer. In some embodiments, the buffer is selected from acetate buffer, histidine buffer, phosphate buffer and succinate buffer, preferably acetate buffer, more preferably acetic acid-sodium acetate buffer.

In an alternative embodiment, the CD40 antibody or the antigen-binding fragment thereof in the pharmaceutical composition has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5.

| Name | Sequence | No. |
|---|---|---|
| HCDR1 | GYILTTYWIT | SEQ ID NO: 3 |
| HCDR2 | DIHPGSGSTKYNEKFKS | SEQ ID NO: 4 |
| HCDR3 | RDY | SEQ ID NO: 5 |
| LCDR1 | RSSQNIVNSQGNTYLE | SEQ ID NO: 6 |
| LCDR2 | KVTNRFS | SEQ ID NO: 7 |
| LCDR3 | FQASLVPWT | SEQ ID NO: 8 |

In alternative embodiments, the CD40 antibody or the antigen-binding fragment thereof in the pharmaceutical composition has a concentration of about 1 mg/ml to 120 mg/ml, which can be about 1mg/ml, 2mg/ml, 3mg/ml, 4mg/ml, 5mg/ml, 6mg/ml, 7mg/ml, 8mg/ml, 9mg/ml, 10mg/ml, 12mg/ml, 14mg/ml, 16mg/ml, 18mg/ml, 20mg/ml, 22mg/ml, 24mg/ml, 26mg/ml, 28mg/ml, 30mg/ml, 35mg/ml, 40mg/ml, 45mg/ml, 50mg/ml, 55mg/ml, 60mg/ml, 65mg/ml, 70mg/ml, 75mg/ml, 80mg/ml, 85mg/ml, 90mg/ml, 95mg/ml, 100mg/ml, 105mg/ml, 110mg/ml, 115mg/ml, 120 mg/ml or any value between any two values, preferably 10 mg/ml to 40 mg/ml, most preferably about 25mg/ml.

In alternative embodiments, the buffer has a concentration of about 5 mM to 30 mM, preferably about 10 mM to 20 mM, and non-limiting examples comprise about 10mM, 12mM, 14mM, 16mM, 18mM, 20mM or any value between any two values, most preferably about 10mM.

In alternative embodiments, the buffer in the pharmaceutical composition has a pH of about 4.5 to 6.5, preferably about 5.0 to 6.0, and in a non-limiting embodiment, it can also be about 5.0, 5.1, 5.2, 5.3, 5.4, 5.5, 5.6, 5.7, 5.8, 5.9, 6.0 or any value between any two values, most preferably about 5.0 or 5.5.

In alternative embodiments, the pharmaceutical composition further comprises sugar. The "sugar" in the present disclosure comprises conventional compositions (CH₂O)ₙ and its derivatives, including monosaccharides, disaccharides, trisaccharides, polysaccharides, sugar alcohols, reducing sugars, non-reducing sugars, and etc. The "sugar" in the present disclosure can be selected from glucose, sucrose, trehalose, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, sorbitol, maltitol, lactitol, iso-maltulose, etc. Preferred sugar is non-reducing disaccharides, more preferably trehalose and sucrose.

In alternative embodiments, the sugar in the pharmaceutical composition has a concentration of about 40 mg/ml to 95 mg/ml (for example, 55 mg/ml), preferably about 60 mg/ml to 90 mg/ml. The concentration of sugar in non-limiting examples comprise 60 mg/ml, 65 mg/ml, 70 mg/ml, 75 mg/ml, 80 mg/ml, 85 mg/ml, 90 mg/ml or any value between any two values, more preferably about 80 mg/ml.

In alternative embodiments, the pharmaceutical composition further comprises a surfactant which can be selected from polysorbate 20; polysorbate 80; poloxamer; Triton; sodium dodecyl sulfonate; sodium lauryl sulfonate; sodium octyl glycoside; lauryl-, myristyl-, linoleyl-, stearyl-sulfobetaine; lauryl-, myristyl-, linoleyl-, stearyl-sarcosine; linoleyl-, myristyl-, cetyl -betaine; lauroamidopropyl-, cocaamidopropyl-, linoleamidopropyl-, myristamidopropyl-, palmamidopropyl-, isostearamidopropyl-betaine; myristamidopropyl-, palmamidopropyl-, isostearamidopropyl-dimethylamine; sodium methylcocoyl; sodium methyloleyl taurate; copolymers of polyethylene glycol; copolymers of polypropylene glycol; copolymers of ethylene glycol and copolymers of propylene glycol, etc. Preferred surfactant is polysorbates, such as polysorbate 80 or polysorbate 20, more preferably polysorbate 80.

In alternative embodiments, the surfactant in the pharmaceutical composition has a concentration of about 0.02 mg/ml to 0.8 mg/ml (for example, 0.1 mg/ml), preferably about 0.3 mg/ml to 0.6 mg/ml. The concentration of the surfactant in non-limiting examples comprise 0.3mg/ml, 0.35mg/ml, 0.4mg/ml, 0.45mg/ml, 0.5mg/ml, 0.55mg/ml, 0.6mg/ml or any value between any two values, more preferably about 0.4mg/ml.

In alternative embodiments, the pharmaceutical composition comprises:
(a) 1 to 120 mg/ml of a CD40 antibody or an antigen-binding fragment thereof, wherein the CD40 antibody or the antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5,
(b) 5 to 30mM of acetate buffer.

In alternative embodiments, the pharmaceutical composition comprises:
(a) 1 to 120 mg/ml of a CD40 antibody or an antigen-binding fragment thereof, wherein the CD40 antibody or the antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5,
(b) 5 to 30mM of acetate buffer, (c) 40 to 90mg/ml of sucrose,
(d) 0.02 to 0.8 mg/ml of polysorbate 80, preferably the pharmaceutical composition has a pH of 4.5 to 6.5, more preferably 5.0 to 6.0, further preferably 5.5.

In alternative embodiments, the pharmaceutical composition comprises:
(a) 1 to 120 mg/ml of a CD40 antibody or an antigen-binding fragment thereof, wherein the CD40 antibody or the antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5,
(b) 5 to 30mM of acetate buffer, (c) 40 to 95mg/ml of trehalose,
(d) 0.02 to 0.8 mg/ml of polysorbate 80, preferably the pharmaceutical composition has a pH of 4.5 to 6.5, more preferably 5.0 to 6.0, further preferably 5.5.

A pharmaceutical composition comprises:
(a) 1 to 120 mg/ml of a CD40 antibody or an antigen-binding fragment thereof, wherein the CD40 antibody or antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5, and
(b) 10 to 20 mM of acetate buffer, and the pharmaceutical composition has a pH of 5.0 to 5.5.

In alternative embodiments, the pharmaceutical composition comprises:
(a) 10 to 40 mg/ml of a CD40 antibody or an antigen-binding fragment thereof, wherein the CD40 antibody or the antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5,
(b) 10 to 20mM of acetate buffer,
(c) 60 to 90mg/ml of sucrose,
(d) 0.3 to 0.8 mg/ml of polysorbate 80, preferably the pharmaceutical composition has a pH of 5.0 to 6.0.

In alternative embodiments, the pharmaceutical composition comprises:
(a) 25 mg/ml of a CD40 antibody or an antigen-binding fragment thereof, wherein the CD40 antibody or the antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5,
(b) 10 to 20mM of acetic acid-sodium acetate buffer,
(c) 60 to 90mg/ml of sucrose,
(d) 0.3 to 0.8 mg/ml of polysorbate 80, preferably the pharmaceutical composition has a pH of 5.0 to 6.0.

In a specific embodiment, the pharmaceutical composition comprises:
25mg/ml CD40 antibody, 80mg/ml sucrose, 0.6mg/ml polysorbate 80 and 10mM histidine-histidine hydrochloride, pH 5.5;
25mg/ml CD40 antibody, 80mg/ml sucrose, 0.6mg/ml polysorbate 80 and 10mM histidine-histidine hydrochloride, pH 5.8;
25mg/ml CD40 antibody, 80mg/ml sucrose, 0.6mg/ml polysorbate 80 and 10mM histidine-histidine hydrochloride, pH 6.0;
25mg/ml CD40 antibody, 80mg/ml sucrose, 0.4mg/ml polysorbate 80 and 10mM histidine-histidine hydrochloride, pH 5.6;
25mg/ml CD40 antibody, 80mg/ml sucrose, 0.4mg/ml polysorbate 80 and 10mM histidine-histidine hydrochloride, pH 5.8;
25mg/ml CD40 antibody, 80mg/ml sucrose, 0.4mg/ml polysorbate 80 and 10mM histidine-histidine hydrochloride, pH 6.0;
25mg/ml CD40 antibody, 80mg/ml sucrose, 0.4mg/ml polysorbate 80 and 10mM histidine-acetic acid buffer, pH 5.0;
25mg/ml CD40 antibody, 80mg/ml sucrose, 0.4mg/ml polysorbate 80 and 10mM histidine-acetic acid buffer, pH 5.5;
25mg/ml CD40 antibody, 80mg/ml sucrose, 0.4mg/ml polysorbate 80 and 10mM histidine-acetic acid buffer, pH 6.0;
25mg/ml CD40 antibody, 80mg/ml sucrose, 0.6mg/ml polysorbate 80 and 10mM histidine-acetic acid buffer, pH 5.0;
25mg/ml CD40 antibody, 80mg/ml sucrose, 0.6mg/ml polysorbate 80 and 10mM histidine-acetic acid buffer, pH 5.5;
25mg/ml CD40 antibody, 80mg/ml sucrose, 0.6mg/ml polysorbate 80 and 10mM histidine-acetic acid buffer, pH 5.8;
25mg/ml CD40 antibody, 80mg/ml sucrose, 0.6mg/ml polysorbate 80, and 10mM histidine-acetic acid buffer, pH 6.0;
25mg/ml CD40 antibody, 80mg/ml sucrose, 0.4mg/ml polysorbate 80 and 10mM acetic acid-sodium acetate buffer, pH 5.5;
or, 25mg/ml CD40 antibody, 80mg/ml sucrose, 0.6mg/ml polysorbate 80 and 10mM acetic acid-sodium acetate buffer, pH 5.5;

In the specific embodiments as defined above, the CD40 antibody has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5.

In some embodiments, the CD40 antibody or the antigen-binding fragment thereof of the present disclosure has a heavy chain variable region shown in SEQ ID NO: 1, and a light chain variable region shown in SEQ ID NO: 2

QVQLQQPGADLVKPGASVKMSCKASGYILTTYWITWVKQRPGQGLEWIGDI
HPGSGSTKYNEKFKSKATLTVDTSSSTAYMQLTRLSSEDSAVYYCARRDYWGQGTTL
TVSS
SEQ ID NO: 1

DVLMTQSPLSLPVSLGDQASISCRSSQNIVNSQGNTYLEWYLQKPGESPKLLI
YKVTNRFSGVPDRFSGSGSGTDFTLKISRVEAEDLGVYYCFQASLVPWTFGGGTKLEI
K

SEQ ID NO: 2

In alternative embodiments, the antibody of the antigen-binding fragment in the pharmaceutical composition can be selected from murine antibody, chimeric antibody and humanized antibody, preferably humanized antibody.

In alternative embodiments, light chain FRs on the light chain variable region and heavy chain FRs on the heavy chain variable region of the humanized antibody in the pharmaceutical composition are respectively derived from human germline light chain and heavy chain, or mutant sequences thereof.

Further, heavy chain of the humanized CD40 antibody has a sequence shown in SEQ ID NO: 17 or a variant thereof, and the variant preferably has 0-10 amino acid variations in heavy chain variable region, more preferably mutations at amino acid positions 6 and 8, wherein the amino acids are preferably mutated to I, A or L; light chain of the humanized antibody has a sequence shown in SEQ ID NO: 18 or a variant thereof, and the variant preferably has 0-10 amino acid variations in light chain variable region, more preferably mutations at amino acid positions 2 and 3, wherein the amino acids are preferably mutated to I, V or L.

In alternative embodiments, the heavy chain variable region of the humanized CD40 antibody further comprises heavy chain FRs of human IgG1, IgG2, IgG3 or IgG4 or a variant thereof, preferably comprises heavy chain FRs of human IgG1, IgG2 or IgG4, and more preferably comprises heavy chain FRs of human IgG1 or IgG2.

In alternative embodiments, amino acid sequence of the light chain of the CD40 antibody has at least 85%, 86%, 87%, 88%, 89%, 90%, 91%, 92%, 93%, 94%, 95%, 96%, 97%, 98%, 99% or 100% sequence identity with amino acid sequence of hu9E5 antibody light chain, amino acid sequence of the heavy chain of the antibody has at least 85%, 86%, 87%, 88%, 89%, 90%, 91%, 92%, 93%, 94%, 95%, 96%, 97%, 98%, 99% or 100% sequence identity with amino acid sequence of hu9E5 antibody heavy chain, sequence of hu9E5 antibody light chain is shown in SEQ ID NO: 18, sequence of hu9E5 antibody heavy chain is shown in SEQ ID NO: 17.

### hu9E5 HC

QVQLVQSGAEVKKPGASVKVSCKASGYILTTYWITWVRQAPGQGLEWMGD
IHPGSGSTKYNEKFKSRVTLTVDTSISTAYMELSRLRSEDTAVYYCARRDYWGQGTTV
TVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK

SEQ ID NO: 17

### hu9E5 LC

DIVMTQSPLSLPVTPGEPASISCRSSQNIVNSQGNTYLEWYLQKPGQSPQLLIY
KVTNRFSGVPDRFSGSGSGTDFTLKISRVEAEDVGVYYCFQASLVPWTFGGGTKVEIK
RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 18

The pharmaceutical composition of the present disclosure has sufficient drug stability and can be stored stably for a long term. On the other hand, for the convenience of drug transportation, the pharmaceutical composition of the present disclosure can be further made into a lyophilized preparation.

The present disclosure also provides a method for preparing a lyophilized preparation comprising CD40 antibody, which comprises the step of lyophilizing the pharmaceutical composition as defined above.

In alternative embodiments, in the method for preparing a preparation comprising CD40 antibody, the lyophilization comprises the steps of pre-lyophilizing, primary drying and secondary drying in sequence.

The present disclosure also provides a lyophilized preparation comprising CD40 antibody prepared by the method for preparing a lyophilized preparation comprising CD40 antibody as defined above.

In some embodiments, the lyophilized preparation is stable at 2-8°C for at least 3 months, at least 6 months, at least 12 months, at least 18 months or at least 24 months.

In some embodiments, the lyophilized preparation is stable at 25°C for at least 3 months, at least 6 months, at least 12 months, at least 18 months or at least 24 months.

In some embodiments, the lyophilized preparation is stable at 40°C for at least 7 days, at least 14 days or at least 28 days.

The present disclosure also provides a method for preparing a reconstituted solution of a lyophilized preparation comprising CD40 antibody, which comprises the step of reconstituted the lyophilized preparation as defined above, and the solution used for reconstitution is selected from, but not limited to, water for injection, physiological saline or glucose solution.

The present disclosure also provides a reconstituted solution of a lyophilized preparation comprising CD40 antibody prepared by the method for preparing a reconstituted solution of a lyophilized preparation containing the CD40 antibody as defined above.

The present disclosure further provides a product or kit comprising a container containing any stable pharmaceutical composition described herein. In some embodiments, the glass bottle is an injection vial made of neutral borosilicate glass tube.

The present disclosure also provides a use of the pharmaceutical composition, the lyophilized preparation or the reconstituted solution of the lyophilized preparation as defined above in the preparation of a medicament for the treatment of CD40-related diseases or symptoms, wherein the diseases or symptoms are preferably cancers, and the cancers are selected from lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma and melanoma.

The present disclosure also provides a method for treating and preventing CD40-related diseases or symptoms, which comprises administering a therapeutically effective amount of the pharmaceutical composition, the lyophilized preparation or the reconstituted solution of the lyophilized preparation as defined above to a patient in need, wherein the diseases or symptoms are preferably cancers, which are selected from lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma and melanoma.

The present disclosure also provides a product comprising a container, which contains the pharmaceutical composition, the lyophilized preparation or the reconstituted solution of the lyophilized preparation as defined above.

It should be understood that one, some, or all of the characteristics of the various embodiments described herein can be combined to form other embodiments of the present disclosure. These and other aspects of the present disclosure will be apparent to those skilled in the art. These and other embodiments of the present disclosure are further described in the following detailed description.

### Detailed description of the preferred embodiment

### 1. Definitions

In order to make it easier for those skilled in the art to understand the present disclosure, certain technical and scientific terms are specifically defined below. Unless otherwise clearly defined elsewhere in this document, all other technical and scientific terms used herein have the meaning commonly understood by a person skilled in the art to which this disclosure belongs.

"Buffer" refers to a buffer that can resistant pH changes through the action of its acid-base conjugated components. Examples of buffers that control the pH in an appropriate range include acetate buffers, succinate buffers, gluconate buffers, histidine buffers, oxalate buffers, lactate buffers, phosphate buffers, citrate buffers, tartrate buffers, fumarate buffers, glycylglycine buffers and other organic acid buffers.

"Histidine buffer" is a buffer containing histidine ions. Examples of histidine buffer includes histidine-hydrochloride buffer, histidine-acetate buffer, histidine-phosphate buffer, histidine-sulfate buffer and etc., preferably histidine-hydrochloride buffer. The histidine-hydrochloride buffer is prepared from histidine and hydrochloric acid or histidine and histidine hydrochloride.

"Citrate buffer" is buffer containing citrate ions. Examples of citrate buffer includes citric acid-sodium citrate buffer, citric acid-potassium citrate, citric acid-calcium citrate, citric acid-magnesium citrate and etc. The preferred citrate buffer is citric acid-sodium citrate buffer.

"Succinate buffer" is buffer containing succinate ions. Examples of the succinate buffer includes succinate-sodium succinate buffer, succinate-potassium succinate buffer, succinate-calcium succinate buffer, and etc. The preferred succinate buffer is succinate-sodium succinate buffer.

"Phosphate buffer" is buffer containing phosphate ions. Examples of the phosphate buffer includes disodium hydrogen phosphate-sodium dihydrogen phosphate buffer, disodium hydrogen phosphate-potassium dihydrogen phosphate buffer, and etc. The preferred phosphate buffer is disodium hydrogen phosphate-sodium dihydrogen phosphate buffer.

"Acetate buffer" is buffer containing acetate ions. Examples of acetate buffer includes acetic acid-sodium acetate buffer, acetic acid-histidine buffer, acetic acid-potassium acetate buffer, acetic acid-calcium acetate buffer, acetic acid-magnesium acetate buffer, and etc. The preferred acetate buffer is acetic acid-sodium acetate buffer.

"Pharmaceutical composition" means a mixture containing one or more of the compounds described herein or their physiologically/pharmaceutically acceptable salts or prodrugs and other chemical components, such as physiologically/pharmaceutically acceptable carriers and excipients. The purpose of the pharmaceutical composition is to promote the administration to the organism and facilitate the absorption of the active ingredients to exert their biological activity. In this context, "pharmaceutical composition" and "preparation" are not mutually exclusive.

In the solution form of the pharmaceutical composition described in the present disclosure, unless otherwise specified, the solvent therein is water.

"Lyophilized preparation" refers to a preparation or a pharmaceutical composition obtained by vacuum lyophilizing a pharmaceutical composition or a solution preparation in the liquid or solution form.

As used herein, term "about" means that the index value is within the acceptable error range of the specific value determined by a person of ordinary skill in the art, and the value partly depends on how it is measured or determined (i.e., the limit of the measurement system). For example, "about" can mean standard deviation within 1 or more than 1 in every practice in the art. Alternatively, "about" or "substantially comprising" can mean a range of up to 20%. Furthermore, especially for biological systems or processes, the term can mean at most an order of magnitude or at most 5 times the value. Unless otherwise stated, when a specific value appears in the present application and claims, the meaning of "about" or "substantially comprising" should be assumed to be within the acceptable error range of the specific value.

The pharmaceutical composition described in the present disclosure can achieve a stable effect: the antibody therein substantially retains its physical stability and/or chemical stability and/or biological activity after storage. Preferably, the pharmaceutical composition retains its physical stability, chemical stability and biological activity after storage. The storage period is generally selected based on the predetermined shelf life of the pharmaceutical composition. There are a variety of analytical techniques available for measuring stability of protein, which can measure the stability after storage at a selected temperature for a selected period of time.

A stable pharmaceutical antibody preparation is a preparation in which no significant changes are observed under the following conditions: storing at refrigerated storage temperature (2-8°C) for at least 3 months, preferably 6 months, more preferably 1 year, and even more preferably up to 2 years. In addition, stable liquid preparations include liquid preparations that exhibit desired characteristics after a period of time including storage at 25°C for 1 month, 3 months or 6 months, or storage at 40°C for 1 month. A typical acceptable criterion of stability is as follows: usually no more than about 10%, preferably no more than about 5% of antibody monomers are degraded measured by SEC-HPLC. As determined by visual analysis, the pharmaceutical antibody preparation is colorless or clear to slightly milky white. The concentration, pH and osmolality of the preparations have a variation of no more than ±10%. Truncations of not more than about 10%, preferably not more than about 5% is generally observed, and aggregates of not more than about 10%, preferably not more than about 5% are generally formed.

After a visual inspection of color and/or clarity, or a measurement by UV light scattering, size exclusion chromatography (SEC) and dynamic light scattering (DLS), if the antibody does not show a significant increase in aggregation, precipitation and/or denaturation, then the antibody "retains its physical stability" in the pharmaceutical preparation. Changes in protein conformation can be evaluated by fluorescence spectroscopy (which determines the tertiary structure of the protein) and by FTIR spectroscopy (which determines the secondary structure of the protein).

If the antibody does not show a significant chemical change, then the antibody "retains its chemical stability" in the pharmaceutical preparation. Chemical stability can be assessed by detecting and quantifying proteins with chemically altered form. Degradation processes that often change the chemical structure of proteins include hydrolysis or truncation (evaluated by methods such as size exclusion chromatography and SDS-PAGE), oxidation (evaluated by methods such as peptide mapping combined with mass spectrometry or MALDI/TOF/MS, etc.), deamidation (evaluated by methods such as ion exchange chromatography, capillary isoelectric focusing, peptide mapping, isoaspartic acid measurement, etc.) and isomerization (evaluated by measuring isoaspartic acid content, peptide mapping etc.).

If the biological activity of the antibody at a given time is within a predetermined range of the biological activity exhibited when the pharmaceutical preparation is prepared, then the antibody "retains its biological activity" in the pharmaceutical preparation. The biological activity of an antibody can be determined, for example, by an antigen binding assay.

The term "CD40" refers to cell surface receptor that is a member of the TNF receptor superfamily, also known as TNFRSF5. CD40 is ubiquitously expressed on the surface of dendritic cells, B cells and macrophages and is a molecule required for the production and maintenance of T cell immunity. The term "CD40" includes any variant or isoform of CD40 that is naturally expressed by a cell. The antibodies of the disclosure can be cross-reactive with CD40 from non-human species. Alternatively, the antibody may be human CD40-specific and may not exhibit cross-reactivity with other species. CD40, or any variant or isoform thereof, can be isolated from cells or tissues in which they are naturally expressed, or produced by recombinant techniques using techniques common in the art and described herein. Preferably, the anti-CD40 antibody targets human CD40 with a normal glycosylation pattern.

The amino acids three-letter code and single-letter code used in the present disclosure are described in J. Biol. Chem, 243, p3558 (1968*).*

The "antibody" mentioned in the present disclosure refers to an immunoglobulin, which is a tetrapeptide chain structure composed of two identical heavy chains and two identical light chains connected by interchain disulfide bonds.

In this disclosure, the antibody light chain can further comprise a light chain constant region, which comprises a human or murine κ, λ chain or variants thereof.

In this disclosure, the antibody heavy chain can further comprise a heavy chain constant region, which comprises a human or murine IgG1, IgG2, IgG3, IgG4 or variants thereof.

Variable fragment (Fv region), composed of about 110 amino acids close to the N-terminus of the antibody heavy chains and light chains, varies considerably in its amino acid sequence. Constant region composed of the remaining amino acid sequence close to the C-terminus of the antibody is relatively stable. The variable region comprises three hypervariable regions (HVRs) and four relatively conserved framework regions (FRs). The three hypervariable regions which determine the specificity of the antibody, are also termed complementarity determining region (CDR). Each of the light chain variable region (LCVR) and the heavy chain variable region (HCVR) consists of three CDRs and four FRs, arranged from amino terminus to carboxy terminus in the following order: FR1, CDR1, FR2, CDR2, FR3, CDR3 and FR4. The three CDRs of the light chain refer to LCDR1, LCDR2 and LCDR3; the three CDRs of the heavy chain refer to HCDR1, HCDR2 and HCDR3. The number and position of CDR amino acid residues of the LCVR and HCVR of the antibodies or antigen binding fragments of the disclosure comply with known Kabat numbering criteria (LCDR1-3, HCDR2-3), or comply with Kabat and Chothia numbering system (HCDR1).

The antibodies of the present disclosure include murine antibodies, chimeric antibodies and humanized antibodies, preferably humanized antibodies.

The term "murine antibody" in the present disclosure is a monoclonal antibody targeting to human CD40 prepared according to the knowledge and skills in the art. During the preparation, the test subject is injected with the CD40 antigen, and then the hybridoma expressing the antibody with the desired sequence or functional characteristics is isolated.

The term "chimeric antibody" is an antibody formed by fusing a variable region of a murine antibody with a constant region of a human antibody, which can alleviate the immune response induced by a murine antibody. To construct the chimeric antibody, hybridoma that secretes murine-specific monoclonal antibody is first constructed, then the variable region gene is cloned from the murine hybridoma cell. Subsequently, the constant region gene of the human antibody is cloned as needed. The murine variable region gene and the human constant region gene are ligated into a chimeric gene and then inserted into a human vector, and finally the chimeric antibody molecule is expressed in the eukaryotic or prokaryotic industrial system. In a preferable embodiment of the present disclosure, the light chain of chimeric CD40 antibody or antigen binding fragment thereof may further comprise a light chain constant region of human κ, λ chain or variants thereof. The heavy chain of chimeric CD40 antibody further comprises a heavy chain constant region of human IgG1, IgG2, IgG3 or IgG4 or variants thereof.

The term "humanized antibody", also known as CDR-grafted antibody, refers to an antibody produced by grafting murine CDR sequences into the frameworks of a human antibody variable region, i.e. the framework sequences of an antibody of different type of human germline. It can overcome the intense immune response induced by chimeric antibody carrying a large amount of murine protein components. Such framework sequences can be obtained from public DNA databases or published references that include germline antibody gene sequences. For example, the germline DNA sequences of variable region genes of human heavy chain and light chain can be found in the "VBase" human germline sequence database (available on the website www.mrccpe.com.ac.uk/vbase), as well as in Kabat, EA, etc., Human, 1991 Sequences of Proteins of Immunological Interest, 5th edition. To avoid a decrease in activity caused by reducing the immunogenicity, the human antibody variable region can be subjected to minimal reverse mutation to maintain the activity. The humanized antibodies of the present disclosure also comprise humanized antibodies that are further subjected to affinity maturation for CDR by phage display.

The term "binding to CD40" in the present disclosure refers to the ability to interact with human CD40.

The term "specific binding" refers to as determined by techniques available in the art, such as competitive ELISA, BIACORE^{®} assay or KINEXA^{®} assay. This term is also applicable when, for example, the antigen-binding domain of the antibody of the present disclosure is specific for a particular epitope carried by many antigens, in which case the antibody carrying the antigen-binding domain can specifically bind to multiple antigens that carry the epitope.

The "antigen-binding fragment" in the present disclosure refers to Fab fragment, Fab' fragment, F(ab')2 fragment and scFv fragment that binds to human CD40, and other fragments that can bind to human CD40, which are formed by utilizing VH and VL regions of the antibodies that can bind to human CD40. It comprises one or more CDRs of the antibodies described in the present disclosure selected from SEQ ID NO: 7, 13, 9, 10, 11 and 12. An Fv fragment comprises heavy chain variable region and light chain variable region of the antibody, but does not comprise the constant region, and is the smallest antibody fragment with all antigen binding sites. Generally, an Fv antibody also comprises a polypeptide linker between the VH and VL domains which can form the structure required for binding antigen. Different linkers can also be used to connect two variable regions of antibodies into a polypeptide chain, which is called single chain antibody or single chain Fv (sFv).

The term "epitope" refers to a part of a molecule that can be recognized and bound by an antibody with one or more antigen binding regions of the antibody.

"Conservative modification" or "conservative substitution or replacement" refers to substitution of amino acids in proteins with other amino acid having similar characteristics (e.g., charge, side chain size, hydrophobicity/hydrophilicity, backbone conformation and rigidity, etc.), such that changes can be made frequently without altering the biological activity of the protein. It is known by those skilled in the art that, in general, a single amino acid substitution in a non-essential region of a polypeptide does not substantially alter its biological activity (see, for example, Watson et al. (1987) Molecular Biology of the Gene, The Benjamin/Cummings Pub. Co., Page 224, (4th edition)). In addition, substitutions of structurally or functionally similar amino acids are unlikely to disrupt the biological activity thereof.

"Identity" or "Homology" of an amino acid sequence refers to sequence similarity between two polypeptide sequences. When both positions in the two compared sequences are occupied by the same amino acid residue, for example, if the same position of each of two polypeptides is occupied by the same amino acid, then the molecule is identical at that position. Examples of algorithms suitable for determining the percent of sequence identity and percent of sequence similarity are the BLAST and BLAST2.0 algorithms, which are described in Altschul et al. (1990) J. Mol. Biol. 215:403-410 *and* Altschul et al. (1977) Nucleic Acids Res. 25:3389-3402*.* The software for performing BLAST analysis is publicly available at the National Center for Biotechnology Information (http://www.ncbi.nlm.nih.gov/).

Methods for producing and purifying antibodies and antigen-binding fragments are well known in the prior art, such as Chapters 5-8 and 15, Using Antibodies: A Laboratory Manual published by Cold Spring Harbor. The antibody or the antigen-binding fragment, as defined in the disclosure, is genetically engineered to add one or more human FRs into non-human CDRs. The human FR germline sequence can be obtained by aligning the IMGT human antibody variable region germline gene database and MOE software from ImMunoGeneTics (IMGT) website http://imgt.cines.fr, or from the Journal of Immunoglobulins, 2001ISBN012441351.

The engineered antibodies or antigen binding fragments of the present disclosure can be prepared and purified by conventional methods. For example, the cDNA sequence encoding the heavy chain and light chain can be cloned and recombined into a GS expression vector. CHO cells can be stably transfected by the recombinant immunoglobulin expression vector. As a more recommended method well known in the art, mammalian expression systems will result in glycosylation of antibodies, particularly at the highly conserved N-terminus of the Fc. Stable clones are obtained by expressing antibodies that specifically bind to human antigens. Positive clones are expanded in serum-free medium in a bioreactor to produce antibodies. Culture medium, into which the antibody is secreted, can be purified and collected by conventional techniques. For example, A or G Sepharose FF column with adjusted buffer is used for purification to wash away non-specifically bound components. Then the bound antibodies are eluted by the pH gradient, and the antibody fragments are detected by SDS-PAGE and then collected. The antibodies can be concentrated by filtration in a conventional manner. Soluble mixtures and multimers can also be removed by conventional methods such as molecular sieves and ion exchange. The resulting product needs to be frozen immediately, such as -70 °C, or lyophilized.

When applying to an animal, human, experimental subject, cell, tissue, organ or biological fluid, "administration" and "treatment", refer to contacting an exogenous drug, therapeutic agent, diagnostic agent or composition with animal, human, subject, cell, tissue, organ or biological fluid. "Administration" and "treatment" can refer to, for example, therapeutic, pharmacokinetic, diagnostic, research, and experimental methods. Treatment of the cells includes contacting reagents with the cells, as well as contacting reagents with the fluid, wherein the fluids are in contact with the cells. "Administration" and "treatment" also means treating, for example, cells *in vitro* and *ex vivo* by reagents, diagnostics, binding compositions, or by another type of cells. "Treatment", as it applies to a human, veterinary or research subject, refers to therapeutic treatment, prophylactic or preventative measures, to research and diagnostic applications.

"Therapy" means the administration of a therapeutic agent for internal or external use comprising, e.g., any of composition that can bind compounds of the present disclosure, to a patient having one or more symptoms of the disease for which the therapeutic agent is known to have therapeutic effect. Generally, the therapeutic agent is administered in an amount that effectively alleviates the symptoms of one or more diseases in a subject or population to be treated, for inducing degeneration of such symptoms or inhibiting the progression of such symptoms to any clinically unmeasurable degree. The amount of therapeutic agent (also referred to as "therapeutically effective amount") effective in alleviating the symptoms of any particular disease can vary depending on a variety of factors, such as disease state, age and weight of the patient, and the ability of the drug to elicit a desired effect in the patient. Whether the symptoms of the disease have been alleviated can be assessed by any clinical test method commonly used by a physician or other health care professionals to assess the severity or progression of the condition. Although embodiments of the disclosure (e.g., therapeutic methods or preparations) may not be effective in alleviating disease symptoms for each target, it shall alleviate disease symptoms of targets in a statistically significant number of patients as determined by any statistical test methods known in the art such as Student's t-test, chi-square test, U test based on Mann and Whitney, Kruskal-Wallis test (H test), Jonckheere-Terpstra test, and Wilcoxon test.

An "effective amount" includes an amount sufficient to ameliorate or prevent a symptom or condition of a medical disease. An effective amount also means an amount sufficient to allow or facilitate the diagnosis. An effective amount for a particular patient or veterinary subject can vary depending on the following factors: for example, the condition to be treated, the overall health of the patient, the route and dosage of the administration, and the severity of side effects. An effective amount can be the maximum dose or dosing regimen that avoids significant side effects or toxic effects.

"Tm value" refers to the temperature of protein thermal denaturation, that is, the temperature at which half of the protein is unfolded, when the spatial structure of the protein is destroyed. Therefore, the higher the Tm value, the higher the thermal stability of the protein.

### 2. Examples and Test Examples

The present disclosure is further explained in detail by the following examples. These examples are for illustrative purposes only and are not intended to limit the scope of the present disclosure.

The experimental methods for which specific conditions are not indicated in the examples of the present disclosure are usually in accordance with the conventional conditions; or in accordance with the conditions suggested by the manufacturers of raw material or commodity. The reagents for which specific sources are not indicated are commercially purchased conventional reagents.

The preparation and purification methods of the CD40 antigen and antibody in this application have been described in the patent document with application number WO2018219327, the contents of which can be incorporated herein by its entirety.

### Example 1. Immune antigens, sequences of antigens for screening and preparation thereof

His-tagged human CD40 (h-CD40-his) recombinant protein, Fc-tagged human CD40 (h-CD40-Fc) recombinant protein, his-tagged murine CD40 (m-CD40-his) recombinant protein and his-tagged rhesus CD40 (rhesus-CD40-his) recombinant protein (#CD0-C52H7) were purified commercial protein reagents purchased from Acrobiosystems. The source of each sequence is shown in Table 1. The protein can be used in each experiment of the following examples.

**Table 1. Source of amino acid sequences of recombinant proteins**

| Name | Start and end of amino acid sequences | Genbank accession number |
|---|---|---|
| h-CD40-his | Glu21-Arg193 | AAH12419.1 |
| h-CD40-Fc | Glu21-Arg193 | NP_001241.1 |
| m-CD40-his | Val24-Arg193 | P27512 |
| rhesus-CD40-his | Glu21-Arg193 | NP_001252791.1 |

### Example 2. Preparation of antibody hybridoma

Anti-human CD40 monoclonal antibodies were produced by immunizing mice, which are experimental C57BL/6 mice, female, 6-8 weeks old [Zhao Yan (Suzhou) New Drug Research Center Co., Ltd., animal production license number: 201503259]. Feeding environment: SPF level. The mice were purchased and feed in a laboratory environment for 1 week with 12/12 hours light/dark cycle adjustment at a temperature of 20-25 °C, and humidity of 40-60%. The mice that have adapted to the environment were divided into 2 cages with 5 mice in each cage.

The immune antigen was an Fc-tagged human modified CD40 recombinant protein (h-CD40-Fc, formulated in phosphate buffer to concentration 1 µg/µl). Freund's adjuvant (Sigma, Lot No.: F5881/F5506) was used for emulsification, wherein, Freund's complete adjuvant (CFA) was used for primary immunization, and nucleic acid adjuvant (CpG, Sangon Biotech) and aluminum for injection (Imject Alum), Thermo, Lot No.: PH203866) for remaining boost immunization. The immunization was performed on Day 0, Day 14, Day 28, Day 42, Day 56, and Day 70. Blood was collected on Day 21, Day 35, Day 49, Day 63, and Day 77 for blood test, and the serum of the mice was detected by ELISA to determine the antibody titer in the serum of the mice.

Upon fourth immunization, spleen cell fusion was performed in mice, in which the antibody titer is high and tends to stable in serum. Boost immunization was performed 3 days prior to fusion, and 10 µg of antigen solution formulated with phosphate buffer was injected intraperitoneally (IP) in each mouse. The spleen lymphocytes were fused with myeloma cell Sp2/0 cells (ATCC^{®} CRL-8287TM) using an optimized PEG-mediated fusion step to obtain hybridoma cells, and monoclonal hybridoma cell lines with good *in vitro* activity were selected.

### Example 3. Cloning and sequencing of anti-CD40 antibody

Hybridoma subclones of the antibody screened and identified above were selected, and hybridoma cells of which in logarithmic growth phase were collected. RNA was extracted using Trizol (Invitrogen, 15596-018) according to the instruction of kit, and reversely transcribed (PrimeScriptTM Reverse Transcriptase, Takara, cat # 2680A). Then the resulting cDNA was amplified by PCR using murine Ig-Primer Set (Novagen, TB326 Rev. B 0503), and sent to sequencing company for sequencing. The sequence of the murine antibody was finally obtained.

The heavy chain variable region and light chain variable region sequences of 9E5 are as follows:

### 9E5 HCVR

QVQLQQPGADLVKPGASVKMSCKASGYILTTYWITWVKQRPGQGLEWIGDI
HPGSGSTKYNEKFKSKATLTVDTSSSTAYMQLTRLSSEDSAVYYCARRDYWGQGTTL
TVSS
SEQ ID NO: 1

### 9E5 LCVR

DVLMTQSPLSLPVSLGDQASISCRSSQNIVNSQGNTYLEWYLQKPGESPKLLI
YKVTNRFSGVPDRFSGSGSGTDFTLKISRVEAEDLGVYYCFQASLVPWTFGGGTKLEI
K
SEQ ID NO: 2

CDR sequences of 9E5 are shown in Table 2 below:

**Table 2**

| Name | Sequence | Number |
|---|---|---|
| HCDR1 | GYILTTYWIT | SEQID NO: 3 |
| HCDR2 | DIHPGSGSTKYNEKFKS | SEQID NO: 4 |
| HCDR3 | RDY | SEQID NO: 5 |
| LCDR1 | RSSQNIVNSQGNTYLE | SEQID NO: 6 |
| LCDR2 | KVTNRFS | SEQID NO: 7 |
| LCDR3 | FQASLVPWT | SEQID NO: 8 |

The obtained variable region sequences were grafted to the constant region sequence of human antibody IgG1 respectively to obtain a human-murine chimeric antibody sequence, and the sequence of the chimeric antibody was inserted into the pCP expression vector (purchased from MabSpace biology Co., Ltd) by molecular cloning technique. These sequences were sequenced and identified after amplifying by PCR (the molecular biological operation methods such as molecular cloning are carried out according to the conventional operating conditions, and can be referred to the "Molecular Cloning: A Laboratory Manual" specifically), and the HEK293 cell expression system can be used to obtain human-murine chimeric antibody 9E5-C. Various *in vitro* activity assays were performed to characterize chimeric antibodies purified by MabSelect SuRe (GE Lifesciences) affinity chromatography. The data are shown in Table 3.

**Table 3 In vitro activity of the chimeric antibody**

| Chimeric antibody | Human CD40-his ELISA EC50 (ng/ml) | Human hCD40/hCD40L ELISA blocking IC50 (µg/ml) | HEK293-CD40 cells binding EC50 (µg/ml) | Biacore Affinity K_{D} (nM) |
|---|---|---|---|---|
| 9E5-C | 1.346 | 0.1218 | 0.03333 | 2.68 |
| Pfizer reference (hIgG4) | 5.628 | 0.2583 | 0.01638 | 20.35 |
| Alligator reference (hIgG1) | 3.288 | 0.7233 | 0.39650 | 65.9 |

### Example 4. Humanization of murine antibody

Based on the typical VH/VL CDR structure of the murine antibody 9E5 obtained, the heavy and light chain variable region sequences were compared with the antibody Germline database to obtain human germline templates with high homology. Among them, the human germline light chain framework region is derived from a human κ light chain gene, and the light chain framework region of the antibody of the present invention is preferably a human germline light chain template Vk2-28/JK4 (9E5). The human germline heavy chain framework region is derived from a human heavy chain, and the heavy chain framework region of the antibody of the present invention is preferably a human germline heavy chain template VH1-2/JH6 (9E5), as shown below:

The heavy chain framework region of 9E5 is preferably a human germline heavy chain template IGHV1-2 (SEQ ID NO: 9):

QVQLVQSGAEVKKPGASVKVSCKASGYTFTGYYMHWVRQAPGQGLEWMG
WINPNSGGTNYAQKFQGRVTMTRDTSISTAYMELSRLRSDDTAVYYCAR

The light chain framework region of 9E5 is preferably a human germline light chain template IGkV2-28 (SEQ ID NO: 10):

DIVMTQSPLSLPVTPGEPASISCRSSQSLLHSNGYNYLDWYLQKPGQSPQLLI
YLGSNRASGVPDRFSGSGSGTDFTLKISRVEAEDVGVYYCMQALQTP

The CDRs of the murine antibody were grafted onto the selected humanized templates to replace the humanized variable regions, and then recombined with the corresponding human IgG constant region (preferably the heavy chain is IgG1 and the light chain is κ). Then, based on the three-dimensional structure of the murine antibody, the embedded residues, the residues that have a direct interaction with the CDRs and the residues that have an important influence on the conformation of VL and VH, were subjected to reverse mutation, and the chemically labile amino acid residues of CDRs were optimized to obtain the final humanized molecules.

### hu9E5-H1a (SEQ ID NO: 11):

QVQLVQSGAEVKKPGASVKVSCKASGYTFTTYWITWVRQAPGQGLEWMG
DIHPGSGSTKYNEKFKSRVTMTVDTSISTAYMELSRLRSEDTAVYYCARRDYWGQGT
TVTVSS

### hu9E5-H1b (SEQ ID NO: 12):

QVQLVQSGAEVKKPGASVKVSCKASGYTFTTYWITWVRQAPGQGLEWMG
DIHPGSGSTKYNEKFKSRVTLTVDTSISTAYMELSRLRSEDTAVYYCARRDYWGQGTT
VTVSS

### hu9E5-H1c (SEQ ID NO: 13):

QVQLVQSGAEVKKPGASVKVSCKASGYILTTYWITWVRQAPGQGLEWMGD
IHPGSGSTKYNEKFKSRVTLTVDTSISTAYMELSRLRSEDTAVYYCARRDYWGQGTTV
TVSS

### hu9E5-L1a (SEQ ID NO: 14):

DIVMTQSPLSLPVTPGEPASISCRSSQNIVNSQGNTYLEWYLQKPGQSPQLLIY

KVTNRFSGVPDRFSGSGSGTDFTLKISRVEAEDVGVYYCFQASLVPWTFGGGTKVEIK

### hu9E5-L1b (SEQ ID NO: 15):

DVVMTQSPLSLPVTPGEPASISCRSSQNIVNSQGNTYLEWYLQKPGQSPQLLI
YKVTNRFSGVPDRFSGSGSGTDFTLKISRVEAEDVGVYYCFQASLVPWTFGGGTKVEI
K

### hu9E5-L1c (SEQ ID NO: 16):

DVLMTQSPLSLPVTPGEPASISCRSSQNIVNSQGNTYLEWYLQKPGQSPQLLI
YKVTNRFSGVPDRFSGSGSGTDFTLKISRVEAEDVGVYYCFQASLVPWTFGGGTKVEI
K

The final humanized hu9E5 antibody molecules (comprising H1c heavy chain and L1a light chain) were selected via the above expression tests and comparison of the number of reverse mutations of combinations of light chains and heavy chains, and the complete light chains and heavy chains sequences of which are set forth in SEQ ID NOs: 17-18, respectively.

### hu9E5 HC

QVQLVQSGAEVKKPGASVKVSCKASGYILTTYWITWVRQAPGQGLEWMGD
IHPGSGSTKYNEKFKSRVTLTVDTSISTAYMELSRLRSEDTAVYYCARRDYWGQGTTV
TVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK

SEQ ID NO: 17

### hu9E5 LC

DIVMTQSPLSLPVTPGEPASISCRSSQNIVNSQGNTYLEWYLQKPGQSPQLLIY
KVTNRFSGVPDRFSGSGSGTDFTLKISRVEAEDVGVYYCFQASLVPWTFGGGTKVEIK
RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 18

### Example 5. Test data of humanized antibody

The binding activity, blocking activity, and the like of the humanized antibodies hu9E5 of the present invention to human and rhesus CD40 are shown in Table 4.

**Table 4 In vitro activity of humanized hu9E5 antibody**

| | Human CD40-his ELISA EC50 (ng/ml) | Rhesus CD40-his ELISA EC50 (ng/ml) | Human hCD40/hCD40L ELISA blocking IC50(µg/ml) | HEK293-CD40 cell binding EC50(µg/ml) | Biacore affinity K_{D} (M) |
|---|---|---|---|---|---|
| Hu9E5-25 | 1.650 | 1.661 | 0.3084 | 0.13970 | 5.301E-9 |
| Alligator reference (hIgG1) | 1.293 | 1.243 | 0.6471 | 1.36200 | 1.66E-7 |
| Pfizer reference (hIgG4) | 3.976 | 3.561 | 0.3106 | 0.01907 | 2.035E-8 |

### Example 6. Inhibition of anti-CD40 antibody on tumor growth in mice

The normal human peripheral blood was taken and the healthy human PBMCs were isolated by density gradient centrifugation. Monocytes were sorted using the CD14⁺ microbeads kit, and CD14⁺ monocytes were isolated according to the protocol provided with the kit, i.e., 20 µl of Anti-CD14 microbeads were added for each 10⁷ cells and incubated at 4 °C for 15 minutes. Then, the cells were added to a magnetic column, and rinsed for three times. Cells in the magnetic column, that is, CD14⁺ monocytes were collected. The RPMI 1640 medium containing 10 ng/ml IL4 and 100 ng/ml GM-CSF was added to the CD14⁺ monocytes, thereby culturing the monocytes for 6 days (culture method is a common method in the art) to perform induction culture of MoDC cells. RPMI 1640 containing IL-2 was added to the remaining cells, and the suspended cells were collected after culturing (the culture method and the method of collecting the cells are all conventional methods in the art). T cells were sorted using the CD3⁺ microbeads kit. Six days later, MoDC cells and CD3⁺ T cells were collected respectively and mixed with Raji cells (Shanghai Institute of Biotechnology Cell Bank, cultured in RPMI1640 medium containing 10% fetal bovine serum) at a ratio of 1:5:20, and inoculated into each NOG mouse (Nanjing Galaxy Biopharma, adaptive feeding for 5 days) subcutaneously. The experimental animals were kept in an independent ventilated box with constant temperature and humidity. The temperature of the breeding room was 18.0-26.0 °C, the humidity was 40-70%, the ventilation was performed for 10-20 times/hour, and the day and night was switched by 12h/12h.

The experimental groups include human IgG1 antibody control group, hu9E5 and reference antibody G12, all administered at a dose of 3 mg/kg. Five mice in each group were administered once a week for six weeks with three consecutive administrations. During the whole experiment, the long-axis diameter and the broad-axis diameter of the tumor were measured twice a week using a vernier caliper, and the tumor volume (mm³) = 0.5 × (tumor long-axis diameter × tumor broad-axis diameter ²) was calculated. Relative tumor inhibition rate TGI (%) was calculated by the following equation: TGI% = (1-T/C) × 100%. T/C % is the relative tumor growth rate, that is, the percentage of relative tumor volume or tumor weight of the treatment group and the control group at a certain time point. T and C are the tumor volume (TV) or tumor weight (TW) of the treatment group and the IgG1 control group at a specific time point, respectively.

The results showed that the humanized anti-CD40 antibody hu9E5 had a very significant anti-tumor effect compared to IgG1 control, with near complete tumor elimination after 21 days of administration.

### Exemplary preparation process of antibody pharmaceutical composition (preparation)

The first step: CD40 antibody (such as hu9E5) and a stable preparation were prepared into a preparation bulk comprising CD40 antibody.

After filtering, the bulk was sampled for sterility test. The bulk was then filtered with a 0.22µm PVDF filter element, and the filtrate was collected.

The second step: the filling volume was adjusted to 1.1ml, and the filtrate was filled into a 2ml vial with stoppers. Samples were taken at the beginning, middle and end of filling to measure the difference of filling volume.

The third step: the capping machine was turned on to perform capping with aluminum cap.

The fourth step: the product was confirmed to have no defects such as inaccurate filling by visual inspection. Next, vial label was printed and pasted. Then carton label was printed while carton box is folded. The product was packed. Finally, box label was pasted onto the box.

### Example 7. Screening of pH value of buffer system for anti-CD40 antibody preparation

The following buffers were formulated to prepare an antibody preparation with anti-CD40 antibody concentration of 50 mg/ml, and samples were taken for stability study at high temperature (40°C).
1) 10mM acetic acid-sodium acetate, pH5.0
2) 10mM acetic acid-sodium acetate, pH5.5
3) 10mM succinic acid-sodium succinate, pH5.0
4) 10mM succinic acid-sodium succinate, pH5.5
5) 10mM succinic acid-sodium succinate, pH 6.0
6) 10mM histidine-histidine hydrochloride, pH5.5
7) 10mM histidine-histidine hydrochloride, pH 6.0
8) 10mM histidine -histidine hydrochloride, pH6.5

**Table 5. Screening results of pH for anti-CD40 antibody**

| **No.** | **Conditon** | **SEC% monomers** | **NR CE-SDS main peak%** | **ICE%** | |
|---|---|---|---|---|---|
| | | | | **Acid peak** | **Neutral peak** |
| 1 | M0 | 97.2 | 95.2 | 34.8 | 60.5 |
| | 40°CM1 | 95.8 | 92.4 | 55.7 | 39.1 |
| 2 | M0 | 97.2 | 95.2 | 36.6 | 58.6 |
| | 40°CM1 | 96.1 | 92.8 | 54.1 | 41.8 |
| 3 | M0 | 97.2 | 95.6 | 35.0 | 60.6 |
| | 40°CM1 | 95.6 | 91.5 | 58.7 | 36.5 |
| 4 | M0 | 97.2 | 95.6 | 35.2 | 60.7 |
| | 40°CM1 | 96.1 | 92.0 | 56.8 | 38.3 |
| 5 | M0 | 97.2 | 95.5 | 34.5 | 61.3 |
| | 40°CM1 | 96.2 | 91.6 | 54.8 | 40.4 |
| 6 | M0 | 97.1 | 95.8 | 34.6 | 60.8 |
| | 40°CM1 | 96.4 | 92.5 | 49.2 | 46.8 |
| 7 | M0 | 97.1 | 95.6 | 34.7 | 61.0 |
| | 40°CM1 | 96.6 | 92.1 | 54.5 | 43.0 |
| 8 | M0 | 97.1 | 95.6 | 34.7 | 60.7 |
| | 40°CM1 | 95.9 | 90.6 | 74.0 | 26.0 |

| | | | | | |
|---|---|---|---|---|---|
| Note: M means month | | | | | |

The results show that the optimal pH range for anti-CD40 antibody is 5.0-6.0. In this pH range, the differences in each of SEC and CE between the three buffer systems is not big, and the variation of ICE is within the range of 14.0%-24.1%, which is more stable than the buffer system (Buffer) with pH 6.5.

### Example 8. Screening of surfactants for anti-CD40 antibody preparations

The buffer systems of 10mM histidine-histidine hydrochloride, pH5.5 and 10mM acetic acid-sodium acetate, pH5.5 were selected to prepare anti-CD40 antibody preparations containing 50mg/ml protein, 75mg/ml sucrose, and different types of surfactants. Stability of their appearances at 25°C was investigated:
1) 10mM histidine-histidine hydrochloride, pH 5.5, containing 0.4mg/ml polysorbate 80;
2) 10mM histidine-histidine hydrochloride, pH5.5, containing 0.4mg/ml polysorbate 20;
3) 10mM acetic acid-sodium acetate, pH 5.5, containing 0.4mg/ml polysorbate 80;
4) 10mM acetic acid-sodium acetate, pH 5.5, containing 0.4mg/ml polysorbate 20;

**Table 6. Screening results of anti-CD40 antibody surfactants at 25°C**

| **No.** | **Condition** | **Appearance** |
|---|---|---|
| 1 | M0 | Clear and colorless |
| | M1 | Occasionally small particles |
| | M3 | Occasionally small particles |
| | M6 | Occasionally small particles |
| 2 | M0 | Clear and colorless |
| | M1 | A few small particles |
| | M3 | Turbid |
| | M6 | Turbid |
| 3 | M0 | Clear and colorless |
| | M1 | Occasionally small particles |
| | M3 | Occasionally small particles |
| | M6 | A few small particles |
| 4 | M0 | Clear and colorless |
| | M1 | A few small particles |
| | M3 | Turbid |
| | M6 | Turbid |

The results show that polysorbate 80 is the preferred surfactant.

### Example 9. Screening of surfactant concentration for anti-CD40 antibody preparations

A buffer system having 10mM of acetic acid-sodium acetate, pH5.5 was selected to prepare anti-CD40 antibody preparations containing 50mg/ml protein, 75mg/ml sucrose, and polysorbate 80 with different concentrations:
1) 10mM acetic acid-sodium acetate, pH5.5, containing 0.1mg/ml polysorbate 80;
2) 10mM acetic acid-sodium acetate, pH 5.5, containing 0.2mg/ml polysorbate 80;
3) 10mM acetic acid-sodium acetate, pH 5.5, containing 0.4mg/ml polysorbate 80;
4) 10mM acetic acid-sodium acetate, pH 5.5, containing 0.6mg/ml polysorbate 80;
5) 10mM acetic acid-sodium acetate, pH 5.5, containing 0.8mg/ml polysorbate 80;

The samples were diluted with 0.9% sodium chloride injection to a protein concentration of 0.5mg/ml, respectively. The results of insoluble particles showed (see Table 3) that when the concentration of polysorbate 80 was 0.1mg/ml~0.8mg/ml, the anti-CD40 antibody protein has good compatibility with sodium chloride, indicating that the polysorbate 80 concentration described above has a good stabilizing effect; then the sample described above was placed in a shaking incubator (25°C, 300rpm) for 5 days. According to the appearance, the concentration of polysorbate 80 is selected to be 0.4 to 0.8 mg/ml, preferably 0.4 mg/ml.

**Table 7 Screening results of polysorbate 80 concentration for anti-CD40 antibody**

| No. | Time | Insoluble particles after dilution (pcs/ml) | | | Appearance after shaking |
|---|---|---|---|---|---|
| | | 2µm | 10µm | 25µm | |
| 1 | 0h | 122 | 5 | 0 | N/A |
| | 2~8°C22h | 287 | 11 | 0 | Turbid |
| 2 | 0h | 112 | 4 | 0 | N/A |
| | 2~8°C22h | 315 | 6 | 0 | Turbid |
| 3 | 0h | 112 | 3 | 0 | N/A |
| | 2~8°C22h | 37 | 1 | 0 | Clear |
| 4 | 0h | 747 | 53 | 2 | N/A |
| | 2~8°C22h | 206 | 20 | 0 | Clear |
| 5 | 0h | 363 | 13 | 0 | N/A |
| | 2~8°C22h | 258 | 10 | 0 | Clear |

### Example 10. Screening of sugar concentration for anti-CD40 antibody preparations

A buffer system having 10mM of acetic acid-sodium acetate, pH5.5 was selected to prepare anti-CD40 antibody preparations containing 50mg/ml protein, 0.4mg/ml polysorbate 80, and sucrose with different concentrations:
1) 10mM acetic acid-sodium acetate, pH5.5, containing 55mg/ml sucrose;
2) 10mM acetic acid-sodium acetate, pH 5.5, containing 65mg/ml sucrose;
3) 10mM acetic acid-sodium acetate, pH 5.5, containing 70mg/ml sucrose;
4) 10mM acetic acid-sodium acetate, pH 5.5, containing 75mg/ml sucrose;
5) 10mM acetic acid-sodium acetate, pH 5.5, containing 80mg/ml sucrose;

The osmotic pressure results of each sample show (see Table 8) that the preparation is isotonic when the sucrose concentration is 80 mg/ml.

**Table 8 Osmotic pressure of anti-CD40 antibody preparation with different sucrose concentrations**

| No. | Sucrose concentration | Osmotic pressure (mOsm) |
|---|---|---|
| 1 | 55mg/ml | 208 |
| 2 | 65mg/ml | 250 |
| 3 | 70mg/ml | 269 |
| 4 | 75mg/ml | 283 |
| 5 | 80mg/ml | 294 |

### Example 11. Stability of different buffer systems for anti-CD40 antibody

The following buffer systems were used to prepare an anti-CD40 preparations containing 50 mg/ml protein, 80 mg/ml sucrose and 0.4 mg/ml polysorbate 80, and the stability at 25°C and 2-8°C was investigated.
1) 10mM acetic acid-sodium acetate, pH5.5
2) 10mM histidine- histidine hydrochloride, pH5.5
3) 10mM histidine-acetic acid, pH5.5

**Table 9 Stability of different buffer systems at 25°C**

| No. | Storage period | Appearance | SEC % | | ICE % | |
|---|---|---|---|---|---|---|
| | | | Polymers | Monomers | Acid peak | Main peak |
| 1 | M0 | Clear and colorless | 0.0 | 97.5 | 32.8 | 64.1 |
| | M3 | Clear and colorless | 0.5 | 96.8 | 41.3 | 56.7 |
| | M6 | Clear and colorless | 1.1 | 96.1 | 47.8 | 49.1 |
| 2 | M0 | Clear and colorless | 0.0 | 97.5 | 32.5 | 64.3 |
| | M3 | Clear and colorless | 0.4 | 96.8 | 40.5 | 57.8 |
| | M6 | Clear and colorless | 0.8 | 95.6 | 50.5 | 46.2 |
| 3 | M0 | Clear and colorless | 0.0 | 97.6 | 31.2 | 65.0 |
| | M3 | Clear and colorless | 0.4 | 96.7 | 38.9 | 59.2 |
| | M6 | Clear and colorless | 0.8 | 95.7 | 52.1 | 44.9 |

**Table 10 Stability of different buffer systems at 2-8°C**

| No. | Storage period | Appearance | SEC % | | ICE % | |
|---|---|---|---|---|---|---|
| | | | Polymers | Monomers | Acid peak | Main peak |
| 1 | M0 | Clear and colorless | 0.0 | 97.5 | 32.8 | 64.1 |
| | M6 | Clear and colorless | 0.6 | 97.9 | 35.2 | 61.3 |
| 2 | M0 | Clear and colorless | 0.0 | 97.5 | 32.5 | 64.3 |
| | M6 | Clear and colorless | 0.5 | 97.9 | 34.8 | 62.1 |
| 3 | M0 | Clear and colorless | 0.0 | 97.6 | 31.2 | 65.0 |
| | M6 | Clear and colorless | 0.5 | 98.1 | 34.8 | 62.0 |

The results showed that after 6 months of storage at 25°C, the 10mM acetic acid-sodium acetate buffer system was slightly superior to the other buffer systems, while the difference between 10mM histidine-histidine hydrochloride and 10mM histidine-acetic acid buffer system was not significant; after 6 months of storage at 2-8°C, there was no significant difference in the quality of the three systems.

### Example 12. The influence of the protein concentration of the preparation on the stability

A buffer system having 10mM of histidine-hydrochloride, pH5.5 was selected to prepare CD40 antibody preparations containing 80mg/ml sucrose, 0.4 mg/ml polysorbate 80 and protein concentrations of 50mg/ml, 25mg/ml and 1mg/ml. The high temperature stability at 40°C was investigated.
1) 10mM histidine-histidine hydrochloride, pH5.5, with a protein concentration of 50mg/ml
2) 10mM histidine-histidine hydrochloride, pH5.5, with a protein concentration of 25mg/ml
3) 10mM histidine-histidine hydrochloride, pH5.5, with a protein concentration of 1mg/ml

**Table 11 High temperature stability of samples with different protein concentrations at 40°C**

| Batch No. | Protein concentration (mg/ml) | Condition | Appearance | SEC% | | | NR CE -SDS% main peak |
|---|---|---|---|---|---|---|---|
| | | | | Polymers | Monomers | Fragments | |
| 1 | 50 | 0h | Clear and colorless | 0.0 | 97.2 | 2.8 | 97.8 |
| | | 40°CM1 | Clear and colorless | 0.5 | 96.1 | 3.4 | 92.5 |
| 2 | 25 | 0h | Clear and colorless | 0.0 | 97.2 | 2.8 | 97.8 |
| | | 40°CM1 | Clear and colorless | 0.4 | 96.0 | 3.7 | 92.9 |
| 3 | 1 | 0h | Clear and colorless | 0.0 | 95.3 | 4.7 | 98.1 |
| | | 40°CM1 | Clear and colorless | 0.6 | 92.6 | 6.8 | 87.4 |

The results show that the protein concentration of the preparations will affect the purity, the protein concentrations of 50mg/ml and 25mg/ml has little effect on the purity, and the stability of the preparation with protein concentration of 1mg/ml is obviously low.

### Example 13. Comprehensive screening of preparation ingredients

In order to further optimize the protein concentration, polysorbate 80 concentration and pH, JMP software was used for DOE design, and RSM model was applied to obtain a series of prescriptions (see below). Since there was no particularly significant difference between the stability data of the buffer systems histidine-histidine hydrochloride (His-HCl) and histidine-acetic acid (His-AA), and the pH buffer range of His-HCl is above 5.5, His-AA was chosen as the buffer for DOE screening experiment. In a buffer system having 10mM histidine-acetic acid (His-AA), anti-CD40 antibody preparations with different protein concentrations, different polysorbate 80 concentrations and 80mg/ml sucrose were prepared, and the samples were stored at 40°C for stability analysis:
(1) 0.4mg/ml polysorbate 80, pH5.0, containing 40mg/ml CD40
(2) 0.2mg/ml polysorbate 80, pH5.5, containing 10mg/ml CD40
(3) 0.4mg/ml polysorbate 80, pH5.5, containing 25mg/ml CD40
(4) 0.4mg/ml polysorbate 80, pH5.5, containing 25mg/ml CD40
(5) 0.4mg/ml polysorbate 80, pH5.0, containing 10mg/ml CD40
(6) 0.6mg/ml polysorbate 80, pH 6.0, containing 40mg/ml CD40
(7) 0.2mg/ml polysorbate 80, pH5.0, containing 25mg/ml CD40
(8) 0.2mg/ml polysorbate 80, pH 6.0, containing 40mg/ml CD40
(9) 0.6mg/ml polysorbate 80, pH5.0, containing 25mg/ml CD40
(10) 0.4mg/ml polysorbate 80, pH5.5, containing 40mg/ml CD40
(11) 0.6mg/ml polysorbate 80, pH5.5, containing 10mg/ml CD40
(12) 0.4mg/ml Polysorbate 80, pH 6.0, containing 10mg/ml CD40

**Table 12 Experimental results of DOE screening at 40°C M1**

| **Batch No.** | **Condition** | **Appearance** | **SEC-monomers %** | **NRCE-SDS Main peak %** | **ICE-Neutral peak %** |
|---|---|---|---|---|---|
| 1 | 0h | Clear and colorless | 95.7 | 97.8 | 71.3 |
| | 40°CM1 | Clear and colorless | 94.2 | 92.1 | 40.5 |
| 2 | 0h | Clear and colorless | 96.2 | 97.9 | 71.5 |
| | 40°CM1 | Clear and colorless | 94.5 | 92.4 | 41.2 |
| 3 | 0h | Clear and colorless | 96.1 | 97.9 | 70.9 |
| | 40°CM1 | Clear and colorless | 94.6 | 91.7 | 41.2 |
| 4 | 0h | Clear and colorless | 96.1 | 98.0 | 72.8 |
| | 40°CM1 | Clear and colorless | 94.5 | 91.0 | 40.3 |
| 5 | 0h | Clear and colorless | 96.5 | 97.9 | 70.4 |
| | 40°CM1 | Clear and colorless | 94.5 | 91.2 | 37.3 |
| 6 | 0h | Clear and colorless | 96.2 | 98.0 | 71.3 |
| | 40°CM1 | Clear and colorless | 95.0 | 91.0 | 40.7 |
| 7 | 0h | Clear and colorless | 96.4 | 97.9 | 72.6 |
| | 40°CM1 | Clear and colorless | 94.8 | 91.1 | 40.6 |
| 8 | 0h | Clear and colorless | 96.2 | 98.0 | 72.3 |
| | 40°CM1 | A few particles | 93.7 | 90.9 | 42.7 |
| 9 | 0h | Clear and colorless | 96.4 | 98.1 | 72.3 |
| | 40°CM1 | Clear and colorless | 94.8 | 90.5 | 40.7 |
| 10 | 0h | Clear and colorless | 96.2 | 98.0 | 70.5 |
| | 40°CM1 | Clear | 95.2 | 90.8 | 42.0 |
| 11 | 0h | Clear and colorless | 96.2 | 98.0 | 73.1 |
| | 40°CM1 | Clear and colorless | 95.3 | 91.3 | 41.0 |
| 12 | 0h | Clear and colorless | 96.3 | 97.9 | 70.1 |
| | 40°CM1 | Clear and colorless | 95.0 | 91.2 | 37.8 |

The results show that: when the pH value was between 5.0 and 6.0, the polysorbate 80 was between 0.2 and 0.6 mg/ml and the protein concentration was between 10 and 40 mg/ml, the fluctuation of the above parameters did not have a great influence on the stability. The pH range of the prescription was 5.0 ~6.0, so the median value of 5.5 was taken as the final pH value, and 0.4mg/ml was taken as the final concentration of polysorbate 80 with overall consideration of previous screening results, and the protein concentration was set as 25mg/ml.

### Example 14. Lyophilization stability of anti-CD40 antibody in different buffer systems

Since the acetic acid (sodium acetate) buffer system may shift the pH value after reconstitution due to volatilization during lyophilization, it is not suitable for use as a buffer system for lyophilized preparations. Two kinds of buffers, such as 10mM histidine-histidine hydrochloride (His-HCl), pH5.5, and 10mM histidine-acetic acid (His-AA), pH5.5, were selected to prepare anti-CD40 antibody preparations containing 50mg/ml protein, 80mg/ml sucrose and 0.4 mg/ml polysorbate 80. The filling volume was 1.2ml/bottle, and the lyophilization process is as shown in Table 13:
1) 10mM histidine-histidine hydrochloride, pH5.5
2) 10mM histidine-acetic acid, pH5.5

**Table 13 Lyophilization process**

| | Temperature | Setting time | Retention time | Vacuum |
|---|---|---|---|---|
| pre-lyophilizing | 5°C | 10min | 1h | / |
| | -45°C | 60min | 2h | / |
| primary drying | -27°C | 90min | 40h | 0.1mbar |
| secondary drying | 25°C | 60min | 6h | 0.01mbar |

The lyophilized product had good cake shape, appearance was clear after reconstitution, and no obvious changes in pH and purity were observed, indicating that the lyophilization process was good. The results show that: two kind of buffers, such as 10mM histidine-histidine hydrochloride, pH5.5, and 10mM histidine-acetic acid, pH5.5, were both suitable for use as buffer systems for anti-CD40 antibody preparations.

**Table 14 Comparison before and after lyophilization**

| Batch No. | Sampling time | Reconstitution time | Appearance | PH | SEC % | | NRCE -SDS Main peak% | ICE % | |
|---|---|---|---|---|---|---|---|---|---|
| | | | | | Polymers | Monomers | | Acid peak | Neutral peak |
| 1 | Before lyophilization | | Clear and colorless | 5. 7 | 0.1 | 99.7 | 95.3 | 35.9 | 61.2 |
| | After lyophilization | <1min | Clear and colorless | 5. 8 | 0.0 | 99.7 | 95.3 | 36.0 | 60.4 |
| 2 | Before lyophilization | | Clear and colorless | 5. 8 | 0.1 | 99.7 | 95.0 | 36.9 | 60.4 |
| | After lyophilization | <1min | Clear and colorless | 5. 9 | 0.1 | 99.9 | 95.0 | 36.4 | 60.5 |

**Table 15 Stability of lyophilization samples at 25°C**

| Batch No. | Sampling time | Appearance | SEC | | | ICE | | |
|---|---|---|---|---|---|---|---|---|
| | | | Polymers | Monomers | Fragments | Acid peak | Neutral peak | Alkaline peak |
| 1 | T0 | Clear | 0.0 | 98.0 | 1.9 | 36.0 | 60.4 | 3.6 |
| | M3 | Small particles | 0.1 | 94.4 | 5.5 | 36.1 | 60.7 | 3.3 |
| 2 | T0 | Clear | 0.1 | 98.0 | 1.9 | 36.4 | 60.5 | 3.1 |
| | M3 | Small particles | 0.1 | 95.1 | 4.8 | 36.0 | 61.1 | 2.9 |

**Table 16 Stability of lyophilization samples at 2-8°C**

| Batch No. | Sampling time | Appearance | SEC | | | ICE | | |
|---|---|---|---|---|---|---|---|---|
| | | | Polymers | Monomers | Fragments | Acid peak | Neutral peak | Alkaline peak |
| 1 | T0 | Clear | 0.0 | 98.0 | 1.9 | 36.0 | 60.4 | 3.6 |
| | M3 | Clear | 0.1 | 94.6 | 5.3 | 38.0 | 59.0 | 3.0 |
| 2 | T0 | Clear | 0.1 | 98.0 | 1.9 | 36.4 | 60.5 | 3.1 |
| | M3 | Small particles | 0.3 | 95.1 | 4.6 | 35.6 | 61.0 | 3.5 |

### Example 15. Other alternative preparations

In addition, the present invention also provides anti-CD40 antibody pharmaceutical preparations with other preparations, including but are not limited to:
(1) 25mg/ml CD40 antibody, 80mg/ml sucrose, 0.6mg/ml polysorbate 80 and 10mM histidine-histidine hydrochloride, pH 5.5;
(2) 25mg/ml CD40 antibody, 80mg/ml sucrose, 0.6mg/ml polysorbate 80 and 10mM histidine-histidine hydrochloride, pH 5.8;
(3) 25mg/ml CD40 antibody, 80mg/ml sucrose, 0.6mg/ml polysorbate 80 and 10mM histidine-histidine hydrochloride, pH 6.0;
(4) 25mg/ml CD40 antibody, 80mg/ml sucrose, 0.4mg/ml polysorbate 80 and 10mM histidine-histidine hydrochloride, pH 5.6;
(5) 25mg/ml CD40 antibody, 80mg/ml sucrose, 0.4mg/ml polysorbate 80 and 10mM histidine-histidine hydrochloride, pH 5.8;
(6) 25mg/ml CD40 antibody, 80mg/ml sucrose, 0.4mg/ml polysorbate 80 and 10mM histidine-histidine hydrochloride, pH 6.0;
(7) 25mg/ml CD40 antibody, 80mg/ml sucrose, 0.4mg/ml polysorbate 80 and 10mM histidine-acetic acid buffer, pH 5.0;
(8) 25mg/ml CD40 antibody, 80mg/ml sucrose, 0.4mg/ml polysorbate 80 and 10mM histidine-acetic acid buffer, pH 5.5;
(9) 25mg/ml CD40 antibody, 80mg/ml sucrose, 0.4mg/ml polysorbate 80 and 10mM histidine-acetic acid buffer, pH 6.0;
(10) 25mg/ml CD40 antibody, 80mg/ml sucrose, 0.6mg/ml polysorbate 80 and 10mM histidine-acetic acid buffer, pH 5.0;
(11) 25mg/ml CD40 antibody, 80mg/ml sucrose, 0.6mg/ml polysorbate 80 and 10mM histidine-acetic acid buffer, pH 5.5;
(12) 25mg/ml CD40 antibody, 80mg/ml sucrose, 0.6mg/ml polysorbate 80 and 10mM histidine-acetic acid buffer, pH 5.8;
(13) 25mg/ml CD40 antibody, 80mg/ml sucrose, 0.6mg/ml polysorbate 80 and 10mM histidine-acetic acid buffer, pH 6.0;
(14) 25mg/ml CD40 antibody, 80mg/ml sucrose, 0.4mg/ml polysorbate 80 and 10mM acetic acid-sodium acetate buffer, pH 5.5;
(15) 25mg/ml CD40 antibody, 80mg/ml sucrose, 0.6mg/ml polysorbate 80 and 10mM acetic acid-sodium acetate buffer, pH 5.5;

The experimental results show that the CD40 antibody preparations with the preparations as defined above had good stability and could be applied to the preparation of CD40 antibody medicaments.

## Claims

1. A pharmaceutical composition comprising a CD40 antibody or an antigen-binding fragment thereof, and a buffer, wherein the CD40 antibody or the antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5, respectively; the buffer is preferably selected from acetate buffer, histidine buffer, phosphate buffer and succinate buffer, more preferably acetate buffer, most preferably acetic acid-sodium acetate buffer.

2. The pharmaceutical composition of claim 1, wherein the CD40 antibody or the antigen-binding fragment thereof has a concentration of 1 mg/ml to 120 mg/ml, preferably 10 mg/ml to 40 mg/ml.

3. The pharmaceutical composition of claim 1 or 2, wherein the pharmaceutical composition has a pH of 4.5 to 6.5, preferably 5.0 to 6.0.

4. The pharmaceutical composition of any one of claims 1 to 3, wherein the buffer has a concentration of 5 mM to 30 mM, preferably 10 mM to 20 mM.

5. The pharmaceutical composition of any one of claims 1 to 4, which further comprises a disaccharide selected from trehalose and sucrose.

6. The pharmaceutical composition of claim 5, wherein the disaccharide has a concentration of 40 mg/ml to 95 mg/ml, preferably 60 mg/ml to 90 mg/ml.

7. The pharmaceutical composition of any one of claims 1 to 6, which further comprises a surfactant that is polysorbate, more preferably polysorbate 80.

8. The pharmaceutical composition of claim 7, wherein the surfactant has a concentration of 0.02 mg/ml to 0.8 mg/ml, preferably 0.3 mg/ml to 0.8 mg/ml.

9. A pharmaceutical composition comprising:
(a) 1 to 120 mg/ml of a CD40 antibody or an antigen-binding fragment thereof, wherein the CD40 antibody or the antigen-binding fragment thereof has LCDR1, LCDR2 and LCDR3 shown in SEQ ID NO: 6, SEQ ID NO: 7 and SEQ ID NO: 8, respectively, and HCDR1, HCDR2 and HCDR3 shown in SEQ ID NO: 3, SEQ ID NO: 4 and SEQ ID NO: 5, respectively,
(b) 5 to 30mM of acetate buffer,
(c) 40 to 90mg/ml of sucrose,
(d) 0.02 to 0.8 mg/ml of polysorbate 80, preferably the pharmaceutical composition has a pH of 4.5 to 6.5, more preferably 5.0 to 6.0, further preferably 5.5.

10. A pharmaceutical composition comprising:
(a) 1 to 120 mg/ml of a CD40 antibody or an antigen-binding fragment thereof, wherein the CD40 antibody or antigen-binding fragment thereof has LCDR1, LCDR2 and LCDR3 respectively shown in SEQ ID NO: 6, SEQ ID NO: 7 and SEQ ID NO: 8, and HCDR1, HCDR2 and HCDR3 respectively shown in SEQ ID NO: 3, SEQ ID NO: 4 and SEQ ID NO: 5, and
(b) 10 to 20 mM acetate buffer, and the pharmaceutical composition has a pH of 5.0 to 5.5.

11. The pharmaceutical composition of any one of claims 1-10, wherein the CD40 antibody or the antigen-binding fragment thereof has a heavy chain variable region shown in SEQ ID NO: 1, and a light chain variable region shown in SEQ ID NO: 2.

12. The pharmaceutical composition of claim 11, wherein the CD40 antibody or the antigen-binding fragment thereof is a humanized antibody.

13. The pharmaceutical composition of claim 12, wherein light chain FR sequences on the light chain variable region and heavy chain FR sequences on the heavy chain variable region of the humanized antibody are respectively derived from human germline light chain and heavy chain, or mutant sequences thereof.

14. The pharmaceutical composition of claim 12 or 13, wherein heavy chain of the humanized antibody has a sequence shown in SEQ ID NO: 17 or a variant thereof, and the variant preferably has 0-10 amino acid variations in heavy chain variable region, more preferably mutations at amino acid positions 6 and 8, wherein the amino acids are preferably mutated to I, A or L; light chain of the humanized antibody has a sequence shown in SEQ ID NO: 18 or a variant thereof, and the variant preferably has 0-10 amino acid variations in light chain variable region, more preferably mutations at amino acid positions 2 and 3, wherein the amino acids are preferably mutated to I, V or L.

15. The pharmaceutical composition of any one of claims 12 to 14, wherein the heavy chain variable region of the humanized antibody further comprises heavy chain FRs of human IgG1, IgG2, IgG3 or IgG4 or a variant thereof, preferably comprises heavy chain FRs of human IgG1, IgG2 or IgG4, and more preferably comprises heavy chain FRs of human IgG1 or IgG2.

16. The pharmaceutical composition of any one of claims 1 to 10, wherein amino acid sequence of the light chain of the CD40 antibody has at least 90% sequence identity with sequence shown in SEQ ID NO: 18, amino acid sequence of the heavy chain of the antibody has at least 90% sequence identity with sequence shown in SEQ ID NO: 17.

17. A method for preparing a lyophilized preparation comprising CD40 antibody, which comprises the step of lyophilizing the pharmaceutical composition of any one of claims 1 to 16.

18. A lyophilized preparation comprising CD40 antibody prepared by the method of claim 17.

19. A use of the pharmaceutical composition of any one of claims 1 to 16 or the lyophilized preparation of claim 18 in the preparation of a medicament for treating CD40-related diseases or symptoms, wherein the diseases or symptoms are preferably cancers, the cancers are selected from lymphoma, breast cancer, ovarian cancer, prostate cancer, pancreatic cancer, kidney cancer, lung cancer, liver cancer, gastric cancer, colorectal cancer, bladder cancer, rhabdomyosarcoma, esophageal cancer, cervical cancer, multiple myeloma, leukemia, gallbladder cancer, glioblastoma and melanoma.

20. A product comprising a container, wherein the container contains the pharmaceutical composition of any one of claims 1 to 16 or the lyophilized preparation of claim 18.
